# EUROPEAN PATENT SPECIFICATION

(11) **EP 1 765 337 B1**
(45) Date of publication and mention of the grant of the patent: **27.08.2008**
(21) Application number: 05774136.5
(22) Date of filing: 08.07.2005
(51) Int. Cl.: A61K 31/427, A61K 31/7076, A61K 31/343, A61P 31/18

(54) **COMBINATION OF TENOFOVIR, RITONAVIR AND TMC114**
KOMBINATION AUS TENOFOVIR, RITONAVIR UND TMC114
ASSOCIATION DE TENOFOVIR, RITONAVIR ET DE TMC114

(30) Priority: 08.07.2004 EP 04103256
(43) Date of publication of application: 28.03.2007
(73) Proprietor: Tibotec Pharmaceuticals Ltd., Little Island, Co. Cork (IE)
(72) Inventor: HOETELMANS, Richard, Marinus, Wilhelmus, NL-4661 WS Halsteren (NL)
(74) Representative: Daelemans, Frank F.R.
(86) International application number: PCT/EP2005/053266
(87) International publication number: WO 2006/005720

(56) References cited:
- WO-A-03/049746
- KEARNEY B P ET AL: "Pharmacokinetic drug interaction and long term safety profile of tenofovir DF and lopinavir/ritonavir." ABSTRACTS OF THE INTERSCIENCE CONFERENCE ON ANTIMICROBIAL AGENTS AND CHEMOTHERAPY, vol. 43, 2003, page 37, XP001204388 & 43RD ANNUAL INTERSCIENCE CONFERENCE ON ANTIMICROBIAL AGENTS AND CHEMOTHERAPY; CHICAGO, IL, USA; SEPTEMBER 14-17, 2003
- TABURET A-M ET AL: "Interactions between atazanavir-ritonavir and tenofovir in heavily pretreated human immunodeficiency virus-infected patients" ANTIMICROBIAL AGENTS AND CHEMOTHERAPY 2004 UNITED STATES, vol. 48, no. 6, June 2004 (2004-06), pages 2091-2096, XP001204386 ISSN: 0066-4804
- "VIREAD (tenofovir disoproxil fumarate) Tablets" GILEAD SCIENCES, [Online] August 2003 (2003-08), pages 1-28, XP002310773 Retrieved from the Internet: URL:http://www.iijnet.or.jp/aidsdrugmhw/Ur gent_Info/viread_pi0308.pdf> [retrieved on 2004-12-15] cited in the application
- Fifteenth International AIDS conference Bangkok 07-2004 abstract TuPeB4634 Hoetelmans Pharmacokinetic interaction between TMC114/ritonavir (RTV) and tenofovir in healthy volunteers XP001207719
- DATABASE PHAR 11 July 2004 (2004-07-11), XP002351634 retrieved from STN Database accession no. 20665
- BOFFITO MARTA ET AL: "Pharmacokinetics of saquinavir hard gel/ritonavir (1000/100 mg twice daily) when administered with tenofovir diproxil fumarate in HIV-1-infected subjects." BRITISH JOURNAL OF CLINICAL PHARMACOLOGY. JAN 2005, vol. 59, no. 1, January 2005 (2005-01), pages 38-42, XP002351620 ISSN: 0306-5251 cited in the application
- DE MEYER SANDRA ET AL: "TMC114, a novel human immunodeficiency virus type 1 protease inhibitor active against protease inhibitor-resistant viruses, including a broad range of clinical isolates." ANTIMICROBIAL AGENTS AND CHEMOTHERAPY. JUN 2005, vol. 49, no. 6, June 2005 (2005-06), pages 2314-2321, XP009055736 ISSN: 0066-4804
- 44th Interscience conference on antimicrobial agents and chemotherapy Washington October/November 2004 abstract A-444 Zong J., Chittick G., Blum MR XP001207718 cited in the application

## Description

The present relates to an anti-HIV combination of a reverse transcriptase inhibitor and two protease inhibitors.

### BACKGROUND OF THE INVENTION

Among the anti-HIV drugs which have been developed are those which target the HIV reverse transcriptase (RT) enzyme or protease enzyme, both of which enzymes are necessary for the replication of the virus. Examples of RT inhibitors include nucleoside/nucleotide RT inhibitors (NRTIs) and non-nucleoside RT inhibitors (NNRTIs). Currently, HIV-infected patients are routinely being treated with three-drug combinations. Regimens containing (at least) three NRTIs; two NRTIs in combination with one or two protease inhibitors (PI)(s); or two NRTIs in combination with a NNRTI, are widely used. When two or more PIs are used in these combinations, one of the PIs is often ritonavir, given at a low sub-therapeutic dose, which acts as an effective inhibitor of the elimination of the other PI(s) in the regimen, resulting in maximal suppression of the virus and thereby reducing the emergence of resistance.

Clinical studies have shown that three-drug combinations of these anti-HIV drugs are much more effective than one drug used alone or two-drug combinations in preventing disease progression and death. Numerous studies of drug combinations with various combinations of such drugs have established that such combinations greatly reduce disease progression and deaths in people with HIV infections. The name now commonly given to combinations of anti-HIV drugs is HAART (Highly Active AntiRetroviral Therapy).

A particular nucleotide reverse transcriptase inhibitor that has been found to be effective in anti-HIV therapy is tenofovir which is also used in the form of its prodrug tenofovir disoproxil fumarate.

Tenofovir is an adenosine nucleotide analogue currently commercially available with activity against retroviruses. Tenofovir disoproxil fumarate (tenofovir DF) is a once-daily, orally administered prodrug of the intravenously administered antiviral agent tenofovir (PMPA). For antiviral activity, tenofovir DF needs to be hydrolysed to the ANP analogue and then phosphorylated to the active diphosphate moiety [Arimilli et al Antiviral Chemistry and Chemotherapy 1997, 8:6 (557-564); Fridland et al. Antiviral Research 1997, 34]. After entry in to lymphocytes or macrophages, the prodrug is quantitatively converted to the parent analogue, tenofovir, and phosphorylated to mono- and diphosphate metabolites. The cellular enzymes that are responsible for phosphorylation of this drug are adenylate kinase and nucleoside diphosphate kinase [Robbins et al. Antimicrobial Agents and Chemotherapy 1995, 39 :10 (2304-2308); Robbins et al. Antimicrobial Agents and Chemotherapy 1998, 42 :3 (612-617)]. Unlike other nucleoside analogues, such as zidovudine or stavudine, both of whose phosphorylation is cell cycle-dependent, tenofovir is efficiently phosphorylated in resting as well as cycling peripheral blood lymphocytes [Robbins et al. 1998]. Tenofovir can inhibit HIV-1 replication in different cell types that may target HIV, including primary human blood lymphocytes and macrophages [Perno et al. Antiviral Research 1992 (289-304); Perno et al. Molecular Pharmacology 1996, 50 :2 (359-366)]. The primary target of tenofovir diphosphate is reverse transcriptase (RT). Tenofovir diphosphate is a competitive inhibitor for the incorporation of deoxyadenosine triphosphate into nascent proviral DNA chains. Inhibition of HIV-1 RT by tenofovir diphosphate has an inhibition constant of approximately 0.9 µM, and if the analogue is incorporated into the growing viral DNA chain it may terminate further chain elongation. Tenofovir inhibits viral RT much more effectively than it inhibits cellular DNA polymerases [Suo et al Journal of Biological Chemistry 1998, 273 :42 (2750-2758)]. The concentration required to inhibit the replication of various HIV-1 strains by 50% (EC50) in lymphocyte and macrophage cell types (MT-2, CEM, ACH8) ranges from 0.2 to 10 µM. The antiviral effect is achieved at non-toxic doses of tenofovir (selectivity index ranging from 100 to 2000). Tenofovir DF is currently available as 300 mg tablets to be taken once daily.

Tenofovir is commonly used in combination with other anti-HIV drugs, especially one or more protease inhibitors for example indinavir, atazanavir, ritonavir and ritonavir/lopinavir The latter is a combination of two protease inhibitors which has been found to have a favourable pharmacokinetic profile when used together in that ritonavir, which is an inhibitor of the cytochrome P₄₅₀ cyclooxygenase enzyme, increases the plasma levels of lopinavir. Tanaka et al. (J. Clin. Pharmacy Therap., 1998, 23, 403-416) describe some anti-HIV protease inhibitors whose metabolism may be dependent on isoforms of cytochrome P₄₅₀. Hsu et al. (Clin Pharmacokinet. 1998, 35, 275-291) describes the pharmacokinetics of ritonavir, including its effect on cytochrome P₄₅₀ isoenzymes.

Combinations of tenofovir with protease inhibitors usually result in improved anti-HIV therapeutic efficacy but there may be undesired interactions between the drugs. For example, plasma levels of lopinavir, one of the two-protease inhibitors in the above lopinavir/ritonavir combination, can decrease when the drug is combined with tenofovir. Thus, co-administration of lopinavir/ritonavir (400/100 mg twice times daily for 14 days) and tenofovir (300 mg once daily) was investigated in 21 healthy volunteers. The AUC, Cmax and Cmin of tenofovir increased by 34%, 31% and 29% respectively. The AUC and Cmax of lopinavir both decreased by 15% and there was no change in lopinavir Cmin. The AUC and Cmax of ritonavir decreased by 24% and 28% respectively, with a 7% increase in ritonavir Cmin (Viread SPC, 2002, Gilead Sciences International Ltd. Viread Product Information, 2002, Gilead Science Inc).

A similar effect was observed with another protease inhibitor, namely atazanavir. Co-administration of ritonavir (100 mg once daily, given with atazanavir) and tenofovir (300 mg once daily) was investigated in 10 male HIV+ subjects. In the presence of tenofovir, there were decreases in ritonavir AUC (7011 to 5217 ng/ml.h), Cmax (886 to 642 ng/ml) and Cmin (43 to 39 ng/ml). Atazanavir concentrations were also decreased in the presence of tenofovir (Pharmacokinetic parameters of atazanavir/ritonavir when combined to tenofovir in HIV-infected patients with multiple treatment failures: a sub-study of PUZZLE2-ANRS 107 trial. Taburet AM, Piketty C, Gerard L, et al. 10th Conference on Retroviruses and Opportunistic Infections, Boston, February 2003, Abstract 537).

There have been various other pharmacokinetic studies of combinations of tenofovir disoproxil fumarate, ritonovir and protease inhibitors such as atazanavir or saquinavir as discussed below.

The pharmacokinetics of atazanavir/ritonavir (300/100 mg once daily) with tenofovir disoproxil fumarate (300 mg once daily) were investigated in 10 HIV-1 infected patients. After the addition of tenofovir disoproxil fumarate, both atazanavir and ritonavir exposure were reduced. Atazanavir Cmax decreased from 5233 ± 3033 to 3443 ± 1412 ng/ml (mean ± sd), AUC decreased from 53761 ± 35255 to 39276 ± 23034 ng/ml.h, and Cmin decreased from 862 ± 838 to 577 ± 367 ng/ml (Taburet AM, Piketty C, Chazallon C, et al. Antimicrob Agents Chemother, 2004, 48:2091-2096*)*.

The coadministration of tenofovir disoproxil fumarate (300 mg once daily) was investigated in 18 HIV-1 infected individuals receiving saquinavir hard gel/ritonavir combination (1000/100 mg twice daily). On day 1, 12 h pharmacokinetic profiles for saquinavir and ritonavir were obtained, tenofovir disoproxil fumarate was then added to the regimen and blood sampling repeated at days 3 and 14. Following the addition of tenofovir disoproxil fumarate, saquinavir and ritonavir plasma concentrations were not significantly different compared with day 1. Geometric mean ratios (95% confidence intervals) for the AUC on days 3 and 14 were 1.16 (0.97, 1.59) and 0.99 (0.87, 1.30) for saquinavir and 1.05 (0.92, 1.28) and 1.08 (0.97, 1.30) for ritonavir *(*Boffito M, Back D, Stainsby-Tron M, et al. Br J Clin Pharmacol, 2005, 59: 38-42*)*.

Coadministration of hard gel saquinavir/ritonavir (1000/100 mg twice daily) alone and with tenofovir disoproxil fumarate (300 mg once daily) was studied in 40 healthy subjects. The pharmacokinetics of tenofovir were not substantially effected by saquinavir/ritonavir (Cmin, Cmax and AUC increased by 23%, 15% and 14% respectively). Ritonavir exposure was slightly increased; Cmin, Cmax and AUC increased by 23%, 10% and 11% respectively. Saquinavir Cmin was moderately enhanced (47% increase); Cmax and AUC increased by 22% and 29% respectively. All subjects achieved a SQV Cmin above 100 ng/ml (Zong J, Chittick G, Blum MR, et al. 44th Interscience Conference on Antimicrobial Agents and Chemotherapy, Washington, October/November 2004, *abstract A*-444*)*.

Other pharmacokinetic studies of combinations of tenofovir disoproxil fumarate, ritonovir and either fosamprenavir or atazanavir are described respectively in Abstracts 10 and 16, 6th International Workshop on Clinical Pharmacology of HIV Therapy: April 28-30, 2005, Quebec, Canada.

KEARNEY et al Abstracts of the interscience conference on antimicrobial agents and chemotherapy, vol. 43, 2003, p. 37 describes the pharmakokinetic and long-term safety of concomitant use of tenofovir and lopinavir/ritonavir. When administered with food, lopimavir an ritonavir pharmacokinetics were equivalent when dosed with or without tenofovir. This finding was in contrast with the previous data where drug administration with meals was not documented.

From these studies it is clear that plasma concentrations of lopinavir or atazanavir may be reduced, which can result in a reduction in the efficacy of the relevant component of the combination and are therefore generally undesirable.

Another protease inhibitor which has been identified for clinical use is the compound herein designated TMC114, which is disclosed in international patent specification WO 95/06030 as compound (4), and has the formula

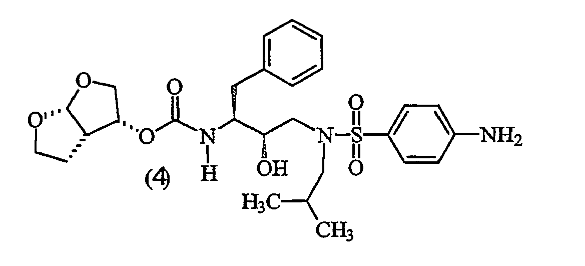

Koh et al (Antimicrobial Agents and Chemotherapy (2003), 7(10), 3123-3129) discuss the antiretroviral activity of TMC114, which is described as a non-peptidic human immunodeficiency virus type 1 (HIV-1) protease inhibitor containing a 3(R),3a(S),6a(R)-bis-tetrahydrofuranylurethane (bis-THF) and a sulfonamide isostere which is extremely potent against laboratory HIV-1 strains and primary clinical isolates (50% inhibitory concentration [IC50], approximately 0.003 µM; IC90, approximately 0.009 µM) with minimal cytotoxicity (50% cytotoxic concentration for CD4+ MT-2 cells, 74 µM).

International patent specification WO 03/049746 describes combinations of a therapeutically effective amount of a hexahydrofuro[2,3-b]furanyl HIV protease inhibitor, including TMC114, and a therapeutically effective amount of a cytochrome P₄₅₀ inhibitor for example ritonavir, ketoconazole, cimetidine or bergamottin.

In view of the reduction in plasma concentrations noted for the above tenofovir/ ritonavir combinations with such protease inhibitors as lopinavir or atazanavir, there is a need for a tenofovir/ ritonavir/protease inhibitor combination where the plasma concentration of the latter protease inhibitor is not reduced to the extent of the combinations noted above.

An object of the invention is to provide a combination of tenofovir with ritonavir and a further protease inhibitor which provides an improved plasma concentration of the latter protease inhibitor compared with the tenofovir/ritonavir/lopinavir combination discussed above.

A further object of the invention is to provide a combination of more than one therapeutically active antiretroviral drug wherein each of the active antiretroviral drugs of the combination can be co-formulated.

Yet a further object of the invention is to provide a combination of more that one therapeutically active antiretroviral drug wherein a therapeutically effective amount of each of the active antiretroviral drugs of the combination can be co-formulated in one single pharmaceutical formulation.

### DESCRIPTION OF THE INVENTION

We have now found that the replacement of lopinavir in the above-described tenofovir/ritononavir/lopinavir combination by the protease inhibitor TMC114, referred to above, results in an improved pharmacokinetic profile.

Thus the present invention provides an anti-HIV combination comprising (i) tenofovir or its disoproxil fumarate derivative; (ii) ritonavir; (iii) and TMC114.

The above combination will be referred to hereinafter as the combination according to the invention.

The use of TMC114 in place of lopinavir has been found to result in an advantageous improvement in terms of the plasma concentration of this protease inhibitor upon administration of the combination.

A further embodiment of the present invention provides for the combination according to the invention for use as a medicine. In another embodiment, the combination according to the invention can be used in the manufacture of a medicament for the treatment or prevention of an HIV infection. A further embodiment provides the use of a combination according to the invention for the manufacture of a medicament for the treatment or prevention of an HIV infection in a human which comprises administering to the human a therapeutically effective amount of the combination according to the invention.

The combination according to the invention is especially useful for the treatment of various HIV infections including AIDS and related clinical conditions such as AIDS related complex (ARC), progressive generalised lymphadenopathy (PGL) or AIDS related neurological conditions such as multiple sclerosis. The combination according to the invention may be particularly useful for the treatment of drug-naïve HIV infected patients.

It will be understood, however, that specific dose level and frequency of dosage of the combination according to the invention for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

In general, for oral administration the components of the combination according to the invention will be administered in the following daily dosages; tenofovir: typically about 300 mg as disoproxil fumarate; ritonavir: generally 100 to 1200 mg, preferably 100 to 400 mg; and TMC114: generally 400 to 1200 mg.

In a preferred embodiment, each of the ingredients of the combination according to the invention can be co-formulated in one pharmaceutical form and do not have to be administered in a separate pharmaceutical form. The daily therapeutic antiretroviral amount of the ingredients of the present combination of such co-formulated single pharmaceutical form may then be given in a single unit dosage form or even in multiple unit dosage forms, such as two, three, four, five or even more unit dosage forms.

Such unit dosage forms unit may contain for example about 300 mg of tenofovir disoproxil fumarate; for example 100 to 400 mg, preferably 100 to 200 mg of ritonavir; and for example 400 to 1200 mg, of TMC114.

Thus, in one embodiment, a pharmaceutical composition is provided comprising a pharmaceutically acceptable carrier and as active ingredients a combination according to the present invention.

The compounds of the combination according to the invention may be administered simultaneously, concurrently or sequentially. Simultaneous administration may be effected by employing a unitary pharmaceutical formulation or separate pharmaceutical formulations. In general, the combination may be administered by the topical, oral, rectal, intravenous, subcutaneous or intramuscular routes. For first line therapy of HIV infection, simultaneous administration employing a unitary pharmaceutical formulation is preferred.

For these purposes, the compositions comprising a combination of the present invention, whether co-formulated in a single formulation or formulated for simultaneous, separate or sequential use, may be administered orally (including suspensions, capsules, tablets, sachets, solutions, suspensions, emulsions, parenterally (including subcutaneous, intravenous, intramuscular, intrasternal injection or infusion techniques), by inhalation spray (including nasal sprays), or rectally (including suppositories)in dosage unit formulations containing conventional non-toxic pharmaceutically acceptable carriers, adjuvants and vehicles.

The present invention also relates to a pharmaceutical composition in a form adapted to be applied to a site where sexual intercourse or related intimate contact can take place, such as the genitals, rectum, mouth, hands, lower abdomen, upper thighs, especially the vagina and mouth, comprising a pharmaceutically acceptable carrier and as active ingredients an effective amount of a combination according to the present invention. As appropriate special adapted compositions there may be cited all compositions usually employed for being applied to the vagina, rectum, mouth and skin such as for example gels, jellies, creams, ointments, films, sponges, foams, intravaginal rings, cervical caps, suppositories for rectal or vaginal application, vaginal or rectal or buccal tablets, mouthwashes. To prepare such pharmaceutical compositions, an effective amount of each of the particular compounds of the combination as the active ingredients is combined in intimate admixture with a pharmaceutically acceptable carrier, which carrier may take a wide variety of forms depending on the form of administration. In order to increase the residence time of such pharmaceutical composition at the site of administration, it may be advantageous to include in the composition a bioadhesive, in particular a bioadhesive polymer. A bioadhesive may be defined as a material that adheres to a live biological surface such as for example a mucus membrane or skin tissue.

Thus, the present invention also relates to a pharmaceutical composition comprising a pharmaceutically acceptable carrier and as active ingredients an effective amount of each of the compounds of the present combination characterized in that the pharmaceutical composition is bioadhesive to the site of application. Preferably, the site of application is the vagina, rectum, mouth or skin, most preferred is the vagina.

### Experimental part

The improved pharmacokinetic profile of the combination according to the invention was demonstrated in the following volunteer study.
**Methods:** 13 healthy volunteers were randomised into 2 cohorts. In session 1, both cohorts received 300 mg TMC114/100 mg ritonavir bid for 6 days, with one dose on day 7, followed by a wash-out period of at least 6 days.
In session 2, both cohorts received 300 mg tenofovir disoproxil fumarate qd for 14 days. In addition, cohort 1 received 300 mg TMC114/100 mg ritonavir bid from day 8 until 14 and cohort 2 received 300 mg TMC114/100 mg ritonavir bid from day 1 until 7. TMC114 was administered as oral solution.

**Results:** Mean plasma concentrations of TMC114 tended to increase in the presence of tenofovir. The increases in LSmean ratios of 24%, 16% and 21% for Cₘᵢₙ, Cₘₐₓ and AUC₁₂ₕ, respectively, were not statistically significant.
Co-administration of tenofovir did not influence the urinary excretion of TMC114. Approximately 7% of the total dose of TMC 114 was excreted unchanged in urine in the presence and absence of tenofovir.
Plasma concentrations of tenofovir were increased when TMC114/ritonavir was co-administered. Based on the ratio of LSmeans, tenofovir Cₘᵢₙ, Cₘₐₓ and AUC₂₄ₕ increased with 37%, 24% and 22%, respectively. These results were statistically significant. The urinary excretion of unchanged tenofovir during one dosing interval was approximately 36% in the presence and absence of TMC114/RTV. Administration of 300 mg TMC114 and 100 mg ritonavir in healthy volunteers with or without tenofovir was well tolerated.

### Conclusions:

The results of this trial demonstrate that the systemic exposure of tenofovir when co-administered with TMC114/ritonavir increased by 22%. Tenofovir did not have a significant influence on TMC114 exposure. It notable that the plasma concentrations of TMC 114 were maintained during exposure to tenofovir compared with a reduction in lopinavir levels noted in the discussion of the prior art.

## Claims

1. An anti-HIV combination comprising (i) tenofovir or its disoproxil fumarate derivative; (ii) ritonavir; and (iii) TMC114.

2. A combination as claimed in claim 1 wherein the components of the combination are in the form of a unitary or separate pharmaceutical compositions.

3. A combination as claimed in claim 1 wherein tenofovir disoproxil fumarate is in a pharmaceutical unit dosage form containing about 300 mg of tenofovir disoproxil fumarate per unit dosage form.

4. A combination as claimed in any of the preceding claims wherein ritonavir is in pharmaceutical unit dosage form containing 100 to 400 mg of ritonavir per unit dosage form.

5. A combination as claimed in any of the preceding claims wherein TMC144 is in a pharmaceutical unit dosage form containing 400 to 1200 mg of TMC114 per unit dosage form.

6. A pharmaceutical composition comprising a combination as claimed in claim 1.

7. A combination as claimed in any of claims 1 to 5 for use as a medicine.

8. A combination as claimed in claim 7 for use in the prevention or treatment of an HIV infection.

9. Use of a combination as claimed in claim 1 for the manufacture of a medicament for the prevention or treatment of an HIV infection.

10. Use of a combination as claimed in claim 1 for the manufacture of a medicament A for the prevention or treatment of an HIV infection in a human, said prevention or treatment comprising administering to said human a therapeutically effective amount of a combination as claimed in claim 1.

11. Use as claimed in claim 10 wherein tenofovir disoproxil fumarate is administered in a daily dose of about 300 mg.

12. Use as claimed in claim 10 wherein ritonavir is administered in a daily dose of 100 to 400 mg.

13. Use as claimed in claim 10 wherein TMC144 is administered in a daily dose of 400 to 1200 mg.

14. Use as claimed in claim 10 wherein the combination is administered in the form of a pharmaceutical composition.

## Patentansprüche

1. Kombination gegen HIV, umfassend (i) Tenofovir oder dessen Disoproxilfumarat-Derivat, (ii) Ritonavir und (iii) TMC114.

2. Kombination nach Anspruch 1, wobei die Bestandteile der Kombination in Form einer einheitlichen pharmazeutischen Zusammensetzung oder getrennter pharmazeutischer Zusammensetzungen vorliegen.

3. Kombination nach Anspruch 1, wobei Tenofovirdisoproxilfumarat in einer pharmazeutischen Dosiseinheit mit etwa 300 mg Tenofovirdisoproxilfumarat pro Dosiseinheit vorliegt.

4. Kombination nach einem der vorhergehenden Ansprüche, wobei Ritonavir in einer pharmazeutischen Dosiseinheit mit 100 bis 400 mg Ritonavir pro Dosiseinheit vorliegt.

5. Kombination nach einem der vorhergehenden Ansprüche, wobei TMC144 in einer pharmazeutischen Dosiseinheit mit 400 bis 1200 mg TMC144 pro Dosiseinheit vorliegt.

6. Pharmazeutische Zusammensetzung, umfassend eine Kombination nach Anspruch 1.

7. Kombination nach einem der Ansprüche 1 bis 5 zur Verwendung als Medizin.

8. Kombination nach Anspruch 7 zur Verwendung bei der Vorbeugung oder Behandlung einer HIV-Infektion.

9. Verwendung einer Kombination nach Anspruch 1 zur Herstellung eines Arzneimittels zur Vorbeugung oder Behandlung einer HIV-Infektion.

10. Verwendung einer Kombination nach Anspruch 1 zur Herstellung eines Arzneimittels zur Vorbeugung oder Behandlung einer HIV-Infektion bei einem Menschen, wobei die Vorbeugung bzw. Behandlung das Verabreichen einer therapeutisch wirksamen Menge einer Kombination nach Anspruch 1 umfaßt.

11. Verwendung nach Anspruch 10, wobei Tenofovirdisoproxilfumarat in einer Tagesdosis von etwa 300 mg verabreicht wird.

12. Verwendung nach Anspruch 10, wobei Ritonavir in einer Tagesdosis von 100 bis 400 mg verabreicht wird.

13. Verwendung nach Anspruch 10, wobei TMC144 in einer Tagesdosis von 400 bis 1200 mg verabreicht wird.

14. Verwendung nach Anspruch 10, wobei die Kombination in Form einer pharmazeutischen Zusammensetzung verabreicht wird.

## Revendications

1. Combinaison anti-VIH comprenant (i) le ténofovir ou son dérivé de fumarate de disoproxil ; (ii) le ritonavir ; et (iii) le TMC114.

2. Combinaison selon la revendication 1, **caractérisée en ce que** les composants de la combinaison sont sous forme de compositions pharmaceutiques unitaires ou distinctes.

3. Combinaison selon la revendication 1, **caractérisée en ce que** le fumarate de disoproxil de ténofovir est sous forme de dose unitaire pharmaceutique contenant environ 300 mg de fumarate de disoproxil de ténofovir par forme de dose unitaire.

4. Combinaison selon l'une quelconque des revendications précédentes, **caractérisée en ce que** le ritonavir est sous forme de dose unitaire pharmaceutique contenant de 100 à 400 mg de ritonavir par forme de dose unitaire.

5. Combinaison selon l'une quelconque des revendications précédentes, **caractérisée en ce que** le TMC144 est sous forme de dose unitaire pharmaceutique contenant de 400 à 1 200 mg de TMC114 par forme de dose unitaire.

6. Composition pharmaceutique comprenant une combinaison selon la revendication 1.

7. Combinaison selon l'une quelconque des revendications 1 à 5, destinée à être utilisée comme médicament.

8. Combinaison selon la revendication 7, destinée à être utilisée dans la prévention ou le traitement d'une infection par le VIH.

9. Utilisation d'une combinaison selon la revendication 1, pour la fabrication d'un médicament destiné à la prévention ou au traitement d'une infection par le VIH.

10. Utilisation d'une combinaison selon la revendication 1, pour la fabrication d'un médicament destiné à la prévention ou au traitement d'une infection par le VIH chez un être humain, ladite prévention ou ledit traitement comprenant l'administration audit être humain d'une quantité thérapeutiquement efficace d'une combinaison selon la revendication 1.

11. Utilisation selon la revendication 10, **caractérisée en ce que** le fumarate de disoproxil de ténofovir est administré en une dose journalière d'environ 300 mg.

12. Utilisation selon la revendication 10, **caractérisée en ce que** le ritonavir est administré en une dose journalière de 100 à 400 mg.

13. Utilisation selon la revendication 10, **caractérisée en ce que** le TMC144 est administré en une dose journalière de 400 à 1 200 mg.

14. Utilisation selon la revendication 10, **caractérisée en ce que** la combinaison est administrée sous forme de composition pharmaceutique.
